# EUROPEAN PATENT APPLICATION

(11) **EP 4 287 202 A1**
(43) Date of publication of application: **06.12.2023**
(21) Application number: 22176648.8
(22) Date of filing: 01.06.2022
(51) Int. Cl.: G16H 30/40, A61C 9/00, G16H 30/20, G06T 7/11, G16H 50/50

(54) **DIGITAL DENTAL IMPRESSIONS**

(71) Applicant: Sirona Dental Systems GmbH, 64625 Bensheim (DE); DENTSPLY SIRONA Inc., York, PA 17401 (US)
(72) Inventor: Maur, Susanne, 64625 Bensheim (DE); Meyer, Marcel, 64625 Bensheim (DE); Wirjadi, Oliver, 64625 Bensheim (DE)
(74) Representative: Özer, Alpdeniz

(57) **Abstract**

A method for optical intraoral imaging of an oral anatomy via an intraoral scanner, comprising: providing initial data related to the oral anatomy and/or the intraoral scanner; computing, using the initial data, a number of intraoral images to be captured via the intraoral scanner; acquiring, via the intraoral scanner, said number of intraoral images at a given location at the oral anatomy; rendering, from at least one of the acquired intraoral images and the initial data or from a plurality of the acquired intraoral images, a more optimally exposed two-dimensional intraoral image. The present teachings also relate to systems, a dental scanner, software products, uses and storage media.

## Description

### TECHNICAL FIELD

The present teachings relate generally to computer-implemented methods and systems for dental scanning and its resulting 3D models.

### BACKGROUND

Dental scanners such as intraoral dental scanners, or simply called dental scanners, are used for capturing digital dental impressions of a patient. An impression is usually in the form of a three-dimensional ("3D") model and may relate to features of interest of oral anatomy, such as teeth and/or soft and/or hard tissues in the patient's oral cavity. The impression may be digitally stored and/or transmitted to a dental practitioner. The digital impression can also be used for other purposes, such as producing a dental restoration and planning or performing a dental treatment.

Most dental scanners are based on structured light principle. Such scanners comprise means for projecting light pattern on the intraoral geometry. The pattern when projected on intraoral geometry appears contoured dependent upon the shape of the geometry. The scanners also comprise a camera for capturing light reflected from the geometries which comprises information related to the contours formed in the projected pattern. Signal from the camera is then sent to a computing unit for extracting shape information of the intraoral geometry. Subsequently, a digital impression or 3D model of the intraoral geometry can be reconstructed from the shape information, which can be in the form of a point cloud, a mesh or alternative shape representations.

US2021267461A1 disclosed an optical measuring method for the three-dimensional detection of the surface of an object using an optical recording unit. The optical recording unit is moved relative to the object during a measurement time interval, height maps are successively detected by the recording unit at a recording frequency, and the detected height maps are each added to an overall height map and displayed.

In recent times, self-scanning or self-use type dental scanners have also been proposed. Such scanners can allow a patient to scan their own oral cavity, or in general allow non-practitioners to perform a scanning operation. This can save costs for the user as compared to having a dental practitioner or professional perform the scan and/or dental health monitoring can be improved by performing more accurate and/or faster scans at home. For direct to customer market, lower cost solutions are generally more attractive.

There is thus recognized a need for improved scanning methods and devices.

### SUMMARY

At least some of the limitations associated with the foregoing can be overcome by the subject matter of the accompanying independent claims. At least some of the additional advantageous alternatives will be outlined in the dependent claims.

The applicant has realized by inventive effort that quality of image acquisition can be made at least partially independent of the light conditions in the oral cavity at the time of acquisition. Advantageously, a number of intraoral images can be traded in for a higher quality of acquisition. More advantageously, a value of the number can be determined prior to performing the acquisition, which can have an advantage that time is saved by capturing just those number of intraoral images which are required for obtaining a pre-determined quality of exposure for a particular acquisition. Another advantage of doing so can be that the requirement of a light source in the intraoral scanner can be at least partially eliminated. In the majority of intraoral scanners, a light source is essential for illuminating the oral anatomy such that intraoral images are captured with a proper exposure. This can however create problems such as unwanted reflections, images with locally overexposed areas, shadows, scattering, and translucency effects in the teeth. Moreover, the light source can cause additional heat production in the scanner as well as increasing the cost and size of the scanner. Especially in optical intraoral imaging such issues may be overpronounced. The present teachings can alleviate these problems.

More specifically, when viewed from a first perspective, there can be provided a computer-implemented method for optical intraoral imaging of an oral anatomy via an intraoral scanner, which method comprises:
- providing initial data related to: the oral anatomy and/or the intraoral scanner;
- computing, using the initial data, a number of intraoral images to be captured via the intraoral scanner;
- acquiring, via the intraoral scanner, said number of intraoral images at or of a given location at the oral anatomy;
- rendering, from at least one of the acquired intraoral images and the initial data or from a plurality of the acquired intraoral images, a more optimally exposed two-dimensional intraoral image.

Thus, a combination of the initial data and at least one of the acquired intraoral images or the plurality of acquired intraoral images can be leveraged to provide the more optimally exposed two-dimensional intraoral image. The rendered image is thus more optimally exposed as compared to any of the intraoral images captured via the intraoral scanner. The present teachings can thus in the minimum sense reduce substantially the requirement of an artificial light source in the intraoral scanner. Thus, a weaker light source may be sufficient to provide the rendered image which is at least similar to a good quality intraoral image captured with a scanner which requires a brighter light source. This can substantially reduce heat produced and thus power consumption of the intraoral scanner. Moreover, the intraoral scanner can be made cheaper and more compact as compared to a scanner with a brighter light source. Another advantage of the present teachings can be that undesired effects associated with bright light sources can be reduced or almost eliminated. For example, in conventional intraoral scanners, bright light from the light source can cause undesired effects such as reflections, translucency in dentition, and shadow effects. Such undesired effects can be avoided at least partially using the present teachings. Additionally, or alternatively, the intraoral scanner may comprise a general-purpose camera, which is not optimized for the dental use. By general-purpose camera it is meant sensors which are used consumer electronics such as a mobile phone, laptop or tablet. The present teachings can thus allow using off-the-shelf components, which can be cost saving in production of such an intraoral scanner. Preferably, the intraoral scanner does not comprise any light source at all. Thus, the intraoral scanner either uses any ambient light available in the oral cavity, or it renders the more optimally exposed 2D intraoral image from poorly exposed intraoral images.

According to an aspect, the more optimally exposed 2D intraoral image or the 2D rendered image, is provided to a Human machine interface ("HMI") device. The user can thus be provided a more realistic image of the oral anatomy despite poor light conditions in the oral cavity. Preferably, a plurality of rendered images is provided to the HMI as a data stream. Hence, realistic video or animation can be provided to the user despite poor lighting in the oral cavity of the patient.

According to an aspect, the method also comprises:
- providing a plurality of the more optimally exposed two-dimensional intraoral images capturing different locations of the oral anatomy;
- reconstructing, using the plurality of the more optimally exposed two-dimensional intraoral images, a three-dimensional digital impression of the oral anatomy.

Thus, a plurality of rendered images, or the more optimally exposed two-dimensional intraoral images, are used to provide the 3D digital impression of the oral anatomy. The reconstruction operation thus analyzes and converts the rendered 2D images to the 3D digital impression, e.g., a surface model or any other suitable form. Hence, a 3D model of the oral anatomy can be provided. The 3D model can thus have reduced gaps or reduced incorrect parts caused due to undesired effects of bright light source. The digital impression is usable for performing a dental procedure and/or for manufacture of a dental object for the patient.

Preferably, the reconstruction of the 3D digital impression is performed via a data-driven reconstruction logic.

"More optimally exposed 2D intraoral image" refers to a 2D color image of a patient's oral cavity characterized by it being of higher quality in terms of noise and/or contrast and/or color rendering and/or brightness and/or sharpness compared to a single image of the same situation produced by a camera using the identical level of illumination.

"More optimally exposed" hence refers to a higher image quality in terms of noise and/or contrast and/or color rendering and/or brightness and/or sharpness compared to the images acquired via the intraoral scanner, especially in challenging environments such as low light environments. Further advantageously, the more optimally exposed 2D intraoral image as generated herein allows alleviation of artifacts introduced by optical properties of the materials visible in the image. Dependent upon lighting conditions, artifacts like translucence and/or reflectance which may be present in a conventional image may be substantially reduced in the more optimally exposed 2D intraoral image.

"Data-driven reconstruction logic" refers to a logic which is trained in a supervised manner using reconstruction training data. According to an aspect, the reconstruction training data may comprise historical rendered images and their corresponding reconstructed digital impression or 3D model. As it was discussed, the data-driven reconstruction logic can reconstruct a 3D surface model from 2D rendered images, which are optical imaging type.

Alternatively, or additionally, the data-driven reconstruction logic may be trained in an unsupervised manner.

The data-driven reconstruction logic is provided with the rendered images as input. The data-driven reconstruction logic provides the digital impression as an output.

"Initial data" in the present context are data related to the oral anatomy and/or data related to the intraoral scanner. Particularly, the initial data comprise information related to the oral anatomy and/or the intraoral scanner which is usable for at least determining light conditions in the oral cavity and/or kinematics of the intraoral scanner at or around the location of the intraoral scanner. Accordingly, the initial data may comprise any one or more of: aperture and/or exposure settings, e.g., of the intraoral scanner, light measurement value (e.g., intensity) at the oral anatomy, kinematic data measured via one or more sensors of the intraoral scanner, and/or data from one or more of the intraoral images which were previously captured in the oral cavity. The sensors may be any one or more of: inertial sensor such as accelerometer and/or gyroscope and/or magnetometer and/or light meter and/or position sensor (absolute and/or relative), and/or proximity sensor.

The initial data are used for determining how many intraoral images are to be captured at a given location at which the intraoral scanner is present in the oral cavity. In some cases, the number may be decided from the initial data, which may or may not be a fixed value. Thus, in some cases, the number of intraoral images captured is the same at different locations in the oral cavity. In other cases, the number may be a dynamic value which changes at different locations at which the intraoral images are captured. Thus, the intraoral scanner may record between one and several intraoral images at a given location in the oral cavity of the patient. Thus, the number of the images is determined from the initial data, or alternatively from a burst parameter in the initial data. "Burst parameter" refers to a pre-defined specification of the number of intraoral images to be captured at a given location which may or may not be part of the initial data and which is used in situations where that number is not computed based on the remaining initial data.

In some cases, the kinematic data may be used for determining how many intraoral images are to be captured and/or for correcting or compensating motion induced artifacts in the one or more of the acquired intraoral images. For example, due to low light conditions in the oral cavity and a long exposure time even a slight movement of the intraoral scanner may introduce artifacts such as blur in one or more of the intraoral images. Pursuant to the present teachings, information related to the movement, e.g., direction and/or type and/or amount of movement, is determined from the kinematic data and corrected or compensated for in the rendered 2D image. Alternatively, or additionally, in some cases, the kinematic data may be used to determine image acquisition settings for capturing the intraoral images such that any artifacts can be avoided or minimized. In some cases, the kinematic data may at least partially be derived by visual odometry via the intraoral scanner.

By saying "*acquiring said number of intraoral images at a given location at the oral anatomy*", it is meant to encompass the images captured at the location with or without the same field-of-view. Thus, it is possible that field-of-view of the intraoral scanner changes during the capture of more than one intraoral image. Small movements in the position and/or angle of the intraoral scanner are thus included in the acquisition at the given location. In other words, those intraoral images which have at least some common imaged portion of the oral anatomy within a given time period can be considered as the intraoral images acquired at a given location at the oral anatomy. The time period may be a few seconds, but in some cases it may be one second or less, e.g., less than half a second. Thus, it may also be said that these intraoral images may be acquired at or around a given location at the oral anatomy.

Thus, according to an aspect, the initial data comprise any one or more of, light intensity measured at the oral anatomy, aperture and/or exposure settings, kinematic data measured via one or more sensors of the intraoral sensor and/or data from previously acquired intraoral images, which may include any one or more of: intraoral images, rendered images, reconstructed digital impression or 3D model.

According to an aspect, at least some of the image acquisition settings of the intraoral scanner are determined using the initial data. The acquisition settings may be any one or more of: exposure time, aperture, focus depth, lens configuration, and sensor sensitivity or gain.

According to another aspect, the rendering of the more optimally exposed two-dimensional intraoral image comprises:
- analyzing each of the acquired intraoral images to select one or more regions of desired exposure;
- combining the selected regions to provide the more optimally exposed two-dimensional intraoral image.

Hence, the acquired intraoral images may be analyzed and scored for quality and/or lightning conditions either as a whole or in parts of the respective intraoral image. High scored intraoral images or parts thereof may be combined to provide the rendered image. For example, each of the images may be divided in parts such as regions or a group of pixels, each part of each image being analyzed or compared with the corresponding part of each other intraoral image. Desired parts, e.g., highest scoring parts, can thus be selected and combined to provide the rendered image. Hence, according to an aspect, the rendering of the more optimally exposed 2D intraoral image comprises adjusting image properties of at least one of the acquired intraoral images and/or at least one of the selected regions. The image properties which are adjusted may be any one or more of: brightness, color, noise, contrast, saturation, intensity, and transformation and/or distortion to compensate for any motion during the burst. The adjustments may thus be made to achieve improvements such as any one or more of: reduction in reflections, reduction in shadows, reduction of overexposure, reduction in translucency effects, achieve harmonization with one or more other intraoral images (e.g., for emphasizing consistency), and differentiation from one or more other intraoral images (e.g., for emphasizing differences). Thus, it may either be combined all pixels of all acquired images with pixel-based weight. Alternatively, just one of the images may be used to select a desired region, then pick corresponding region to the desired region from every other image and combine these regions to provide the optimally exposed two-dimensional intraoral image. Further alternatively, a group or all of acquired images may be used to select desired regions and then said desired regions may be combined to provide the optimally exposed two-dimensional intraoral image.

Preferably, the rendering is at least partially performed via a data-driven rendering logic.

"Data-driven rendering logic" refers to a data-driven logic which is trained in a supervised manner using rendering training data. According to an aspect, the rendering training data comprise a plurality of historical intraoral images and their corresponding rendered image. Preferably, the rendering training dataset comprise a plurality of sets of historical intraoral images and their corresponding historical rendered images. Alternatively, or additionally, the data-driven rendering logic may be trained in an unsupervised manner.

The data-driven rendering logic thus generates the more optimally exposed two-dimensional intraoral image or the rendered image as an output when the acquired intraoral images, and preferably the initial data, are provided at its input. As it was previously discussed, the acquired intraoral images in some cases may be a single image.

In some cases, there may be more than one data-driven logics which are used for performing different operations. Any two or more of the data-driven logics may be combined as one or more combined logics. According to an aspect, all data-driven logics are in the form of the same combined logic which is trained in an end-to-end manner, using end-to-end training data comprising historical upstream inputs and their corresponding desired historical outcomes at the output downstream of the combined logic.

According to an aspect, at least some of the parts or regions are selected via a segmentation logic. The segmentation logic is preferably at least partially a data-driven logic, or the segmentation logic may at least partially be a data-driven segmentation logic. The segmentation logic may perform a segmentation and/or a localization operation on each of the intraoral images.

"Segmentation operation" in the present context refers to a computerized processing operation which concludes with demarcation of at least one anatomical feature and/or intraoral region of the oral anatomy in an intraoral image with a computer-generated feature boundary. The intraoral regions may, for example, be those regions which are typically difficult to capture in imaging. These regions might not only depend on the material (anatomical feature), but also the lightning and viewing angles.

The segmentation logic may perform a segmentation operation for detecting the one or more pre-selected anatomical features. Thus, the segmentation logic may segment each of the intraoral images for detection. Thus, the segmentation logic may extract attributes or one or more objects of interest from at least one of the intraoral images. The segmentation logic may classify every pixel in the respective intraoral image to an object according to its context such that each pixel is assigned to a specific object. Alternatively, or in addition, the segmentation logic may classify every pixel in the respective intraoral image to a class according to its context such that each pixel is assigned to a specific class. A non-limiting example of a segmentation operation when applied using the present teachings may be an outline around a pre-selected anatomical feature, such as a specific tooth or even a group of teeth. Another non-limiting example is an outline around a specific pathology. Said segmented outline corresponds to the shape of the respective anatomical feature as visible in the respective intraoral image.

There may be a plurality of segmented objects in any given intraoral image.

The segmentation logic may comprise an analytical model using any suitable segmentation algorithm, e.g., point and line detection, edge linking, and/or thresholding method, histogram, adaptive, and their likes.

Preferably, the segmentation logic is at least partially a data-driven logic which is trained in a supervised manner using a segmentation training dataset comprising a plurality of pre-segmented historical intraoral images. Alternatively, or additionally, the data-driven segmentation logic is trained in an unsupervised manner. The data-driven segmentation logic thus receives acquired intraoral images as input and it provides segmented intraoral images as output.

In some cases, the segmentation logic or another logic may also perform a localization operation, which may be performed via an analytic part and/or a data-driven part.

A "localization" operation in the present context refers to a computerized processing operation which concludes with a more general region or location within which a specific anatomical feature is located in an intraoral image. The localization may also result in a boundary within which said anatomical feature is located, however, unlike a segmentation operation, a pixel-by-pixel evaluation is not performed. Hence, a localization boundary, if generated, may be broader than a segmented boundary around a given anatomical feature. Additionally, or alternatively, a localization operation may conclude with annotating or tagging a specific anatomical feature and/or intraoral region of the oral anatomy in the intraoral image, for example in any location on said anatomical feature. The intraoral regions may, for example, be those regions which are typically difficult to capture in imaging. These regions might not only depend on the material (anatomical feature), but also the lightning and viewing angles.

When viewed from another perspective, there can also be provided a use of the three-dimensional digital impression as herein generated for performing a medical procedure, such as a dental procedure and/or for manufacture of a dental object and/or for display at an HMI device.

When viewed from yet another perspective, there can also be provided a use of one or more of the more optimally exposed two-dimensional intraoral images as herein generated for reconstructing a digital dental impression and/or display at a human machine interface device and/or training and/or validating a data-driven logic.

When viewed from another perspective, there can also be provided a system comprising means for performing the steps of any of herein disclosed methods.

For example, there can be provided a system comprising an interface for operatively connecting to an intraoral scanner, and one or more computing units, wherein any of the computing units are configured to:
- providing initial data related to: the oral anatomy and/or the intraoral scanner;
- computing, using the initial data, a number of intraoral images to be captured via the intraoral scanner;
- acquiring, via the intraoral scanner, said number of intraoral images at a given location at the oral anatomy;
- rendering, from at least one of the acquired intraoral images and the initial data or from a plurality of the acquired intraoral images, a more optimally exposed two-dimensional intraoral image.

The system may for example be a scanning system or it may be an addon to the scanning system. According to an aspect, the system may be implemented on a distributed system, such as on a cloud-computing platform. The intraoral images and/or the rendered image and/or the digital impression may or may not be provided at the same memory storage. The intraoral scanner may comprise at least one of the computing units and/or it may be operatively connected to some of the computing units in a wired or wireless manner, e.g., via a connectivity interface and/or a network interface. The intraoral images and/or the rendered image and/or the digital impression may or may not be provided at the same memory storage. The memory storage may be local to the intraoral scanner and/or it may be part of a remote or a cloud-based service.

When viewed from another perspective, there can also be provided a computer software product, or a non-transitory computer-readable storage medium storing the program, comprising instructions which when executed by one or more suitable computing units cause any of the computing unis to perform the steps of any of the methods herein disclosed.

There can also be provided a data-storage medium storing thereupon one or more of the more optimally exposed two-dimensional intraoral images and/or the digital impression as herein generated.

"Oral anatomy" refers to the region and anatomical parts in the oral cavity of a patient. Thus, oral anatomy comprises structures such as dentition and/or gingiva and/or other parts. In some cases, at least some of the oral structures may be artificial structures such as one or more dental replacements or prosthetics, e.g., dental crown, bridge, veneer, implant, etc. Alternatively, or in addition, the parts may in some cases even be a scan body (or scanbody) or any other natural and/or artificial structure.

"Anatomical features" or dental features refers to information related to oral structures such as dentition and/or gingiva and/or other features of oral anatomy of a patient. Alternatively, or in addition, at least some of the oral structures of which anatomical features are recorded may be one or more artificial structures such as one or more dental replacements, e.g., dental crown, bridge, veneer, implant, etc. Alternatively, or in addition, the anatomical feature may in some cases even be a scan body (or scanbody) or other natural and/or artificial structure. The anatomical features, usually in the form of surface data, are recorded for building the digital impression of a patient's oral anatomy. Usually for producing the digital impression structures which are rigidly attached to the jaw of the patient are of more interest.

Alternatively, or additionally, the anatomical feature may even be information related to a pathology or condition. As non-limiting examples, pathology or condition may be any one or more of, fracture or crack of tooth or caries, impaction, or any other visually characterizable state of the oral anatomy or any part thereof. A few non-limiting examples of the oral features or dental features are, any one or more of: shape, outline or boundary of any one or a group of teeth, their arrangement, sequence in any direction, a presence or a lack of presence of an expected feature such as a given tooth type. Alternatively, or additionally, the anatomical features may also be a shape, region, or a physical characteristic detected within an area or interest, such as an outline or boundary. Alternatively, or additionally, the anatomical features may also be, or they may be detected via, an interrelation between two different anatomical features. For example, a boundary between a tooth and gingiva.

"Intraoral scanner" refers to a device or a system which is used for acquiring the intraoral images in a patient's oral cavity. The intraoral scanner may either be in the form of a wand or it may comprise a wand part for inserting in the patient's mouth for acquiring the intraoral images. In some cases, the intraoral scanner may be part of an arrangement comprising a camera unit which can be coupled to or attached to another device. The coupling may be a physical attachment, detachable or permanent, or it may be a flexible coupling, e.g., wired or even wireless e.g., via a connectivity interface. The camera unit may be which can be placed inside or in front of the mouth

"Intraoral image" refers to an optical image of the oral anatomy captured in the oral cavity of a patient. The intraoral image is acquired via the intraoral scanner. The intraoral image is an optical or visual light image captured via a camera of the intraoral scanner. The intraoral image may even be an image acquired under low-light conditions.

In some cases, the intraoral images may be in the form of a data-stream provided via the intraoral scanner, or they may be provided via the data-stream.

In the present context, a "digital impression" or "3D model" refers to a digital model obtained via reconstruction using a plurality of rendered images or the more optimally exposed two-dimensional intraoral images of a patient.

The digital impression is a digital surface representation of the patient's oral anatomy. Hence, the 3D model comprises information related to a plurality of anatomical features of the patient's oral anatomy.

The digital impression may include a representation of the patient's teeth and/or hard and/or soft tissues in and around the patient's gums. The digital impression may be usable for providing for the patient any one or more of: dental treatment or procedure, surgical templates, custom devices, prosthetic restorations, and orthodontic aligners. The terms 3D model and digital impression may be used interchangeably in the present disclosure.

"Optical imaging" refers to the process of producing a visual image or photographic image of an object. More specifically, optical imaging refers to imaging which can produce an image which comprises information of natural or near natural colors and/or texture of the object which is imaged. Accordingly, optical imaging may be performed using a white light or a light which allows the resulting visual image to show natural or near natural colors and/or texture to a human. Hence, when an optical image with optimal exposure is shown via the HMI, the user may be provided a naturalistic reproduction of the object, at least in terms of color and/or texture. It shall be understood that for a greyscale visual image, texture may be a more central feature, whereas, for a color image, at least colors should be naturalistic, preferably both color and texture. An optical image may be captured using visual light and/or light outside the visual spectrum. The reproduction of natural colors depends on the light conditions and the material and/or texture of the imaged object and the used sensor properties.

Hence, "optical intraoral imaging" relates to producing an optical image of the oral anatomy of the patient using optical imaging. Accordingly, the rendered image is also an optical intraoral image of the oral anatomy of the patient.

"Structured light" in context of "structured light imaging" refers to projected light which comprises a pre-determined pattern. The structured light or pattern is projected onto a target surface for determining topography of the surface. The surface topography induces distortion in the pattern which is projected onto it. The distortion is recorded via an imaging sensor or camera and further analyzed to extract surface information and eventually build a digital model of the surface. It shall be appreciated that in context of intraoral scanning, the target surface is usually the oral anatomy of a patient. As the dental scanner is moved along the areas or locations adjacent to the surface, the digital impression of the oral anatomy is expanded by stitching together the surface data from different locations. The term "structured light imaging" thus refers to the process of projecting structured light patterns and extracting the surface data from the reflections of said patterns.

"Data-driven logic" refers to a logic, which at least partially derives its functionality from training data. In contrast to a rigorous or analytical logic which is based on programmed instructions for performing a particular logical task, a data-driven logic can allow forming such instructions at least partially automatically using the training data. A data-driven logic may be able to detect patterns or indications (e.g., in input or input data provided to the data-driven logic) which can otherwise not be known or may be difficult to implement as an analytical logic form.

The data-driven logic may be in software and/or hardware form, for example, executable via one or more computing units.

It shall be appreciated that in the present context, the data-driven logic refers to a trained mathematical logic which is parametrized according to the respective training data set. For example, the anatomy data-driven logic is parameterized via its respective training data to detect one or more anatomical features. An untrained logic lacks this information. Hence, the untrained logic or model is incapable for performing a desired detection. Feature engineering and training with the respective training datasets thus enables parametrization of the untrained logic. The result of such a training phase is the respective data-driven model, which as a result of the training process, preferably solely as a result of the training process, provides interrelations and logical capability related to the purpose for which the respective data-driven logic is to be used.

The data-driven logic preferably comprises, or it is, a regression logic. In some cases, the data-driven logic may be combined with or it may further include an analytical logic. The analytical logic may describe the relation between its input and output as a function or one or more mathematical equations or logical criteria. Thus, any of the data-driven logics described herein may even be a hybrid logic. A hybrid logic refers to a logic which comprises first-principles parts, so called white-box described via a set of equations e.g., mathematical equations, as well as data-driven parts as explained previously. The data-driven part may also be called black-box logic or model. Hence, the data-driven logic in some cases may be a combination of white-box logic or analytical logic, and black-box logic. In some cases, the data-driven logic may be, or it may even have a grey-box part. A grey-box logic in this context refers to a logic or model which combines a partial analytical structure with a data-driven part using training data to complete the model.

"Combined logic" refers to a logic comprising at least one of the two different data-driven logics disclosed herein. The combined logic may be pretrained in a supervised manner using an end-to-end training process. The end-to-end training process in this context may refer to at least partially annotated data comprising inputs and their corresponding desired outputs. In some cases, the combined logic may be trained fully or partially in an unsupervised manner.

"Human machine interface" or "HMI" refers to a device or system which comprises at least one video unit or display screen for displaying to a user the acquired intraoral images or the rendered image and/or the reconstructed 3D model.

Thus, the HMI system may comprise a visual display or screen for displaying visual images or video. Optionally, the HMI system may also comprise an audio device. For example, the HMI system may also comprise a loudspeaker for outputting audio. The video unit may comprise a video screen which may be active or passive, for example an LCD screen, OLED screen or projection based video system. Alternatively, or additionally, the video unit may comprise an augmented reality ("AR") device.

The HMI may even comprise one or more input devices for receiving user input.

The terms "computing unit", "computing device", "processing unit" or "processing device" may be used interchangeably in the present disclosure. The computing unit may comprise, or it may be in the form of, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). the processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Network interface" refers to a device or a group of one or more hardware and/or software components that allow an operative connection with the network. For example, the network interface may be used to connect to a remote server or cloud service. In some cases the network interface may be a part of the connectivity interface.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

"Connectivity interface" or "communication interface" refers to a software and/or hardware interface for establishing communication such as transfer or exchange of signals or data. The communication may either be wired, or it may be wireless. The connectivity interface may be used to connect one or more devices, such as a dental scanner to a computing unit and/or a memory storage. Connectivity interface is preferably based on or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long distance protocol. The connectivity interface may support any one or more standards and/or proprietary protocols.

That two or more components are "operatively" coupled or connected shall be clear to those skilled in the art. In a non-limiting manner, this means that there may be at least one communicative connection between the coupled or connected components e.g., they are the connectivity interface, the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a block diagram of a system pursuant to the present teachings;
FIG. 2 illustrates a flowchart 200 showing a method aspect of the present teachings.

### DETAILED DESCRIPTION

In accordance with example aspects described herein, methods, systems and computer readable storage media can be provided, e.g., for optical intraoral imaging of an oral anatomy.

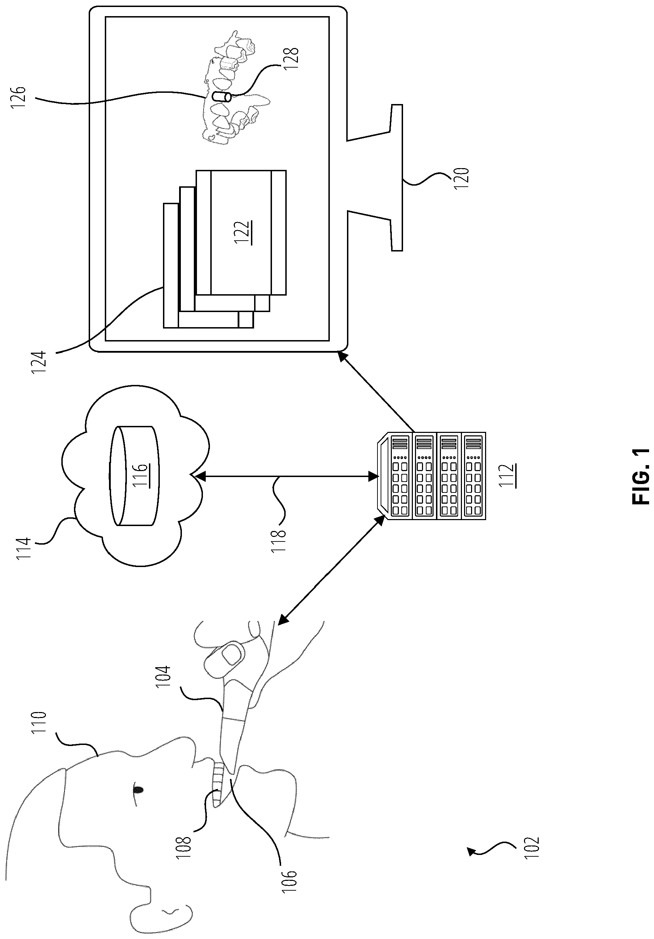

FIG. 1 shows a block diagram 102 of a system illustrating certain aspects of the present teachings. The system comprises an intraoral scanner 104, which is shown in FIG. 1 being used for intraoral scanning of oral anatomy 108 of a patient 110 by introducing in the patient's oral cavity 106. The intraoral scanner 104 is operatively connected to a computing module 112, which comprises at least one computing unit. In some cases, the computing module 112 may be located at least partially within the intraoral scanner 104. In some cases, the computing module 112 may at least partially be a part of a cloud service 114. Thus, the computing module 112 may be a local device at the location of the intraoral scanner 104 and/or at least partially a remotely located device or system, e.g., one or more cloud services 114 and/or a remote server. The computing module 112 is also operatively connected to a memory storage 116, which may at least partially be a part of the cloud service 114. In this case, the memory storage 116 is shown as a part of the cloud service 114. The intraoral scanner 104 may connect to an external computing module 112 via a connectivity interface (not explicitly shown in FIG. 1). Any local devices, e.g., the intraoral scanner 104 and/or the local computing module 112 may connect to the cloud service 114 via one or more networks 118. A network interface (not explicitly shown in FIG. 1) may be used to connect the local devices to the remote devices, e.g., the cloud service 114. In some cases, the network interface and the connectivity interface may be the same unit.

Optionally, the computing module 112 may be operatively connected to an HMI 120, which in FIG. 1 is shown as a computer screen. The connection between the computing module 112 and the HMI 120 may be via an output interface (not explicitly shown in FIG. 1), which may for example be a computer port or bus, e.g., display port, HDMI, USB, or their like. However, the output interface may even be an API for providing data to one or more computing units on the HMI 120 side. In some cases, the output interface may be a part of the network interface and/or the connectivity interface. Accordingly, it is possible that the three interfaces are the same device.

The intraoral scanner 104 comprises a camera (not explicitly shown in FIG. 1) for optically imaging the oral anatomy 108. Optionally, the intraoral scanner 104 may comprise a visual light source for assisting in optical imaging (not explicitly shown in FIG. 1). Pursuant to the aspects of the present teachings the light source can be at least substantially weaker than conventional light sources required for visual light imaging of the oral cavity 106. Preferably, the present teachings completely offset the requirement of the light source. The teachings can in some cases even be applied to conventional visual light imaging intraoral scanners where a deployment of the light source can be controlled via the computing unit such that the light source is only used in substantially dark or completely dark conditions.

The computing module 112 determines or computes from initial data, how many intraoral images are to be captured via the intraoral scanner 104, or more specifically via the camera. At least a part of the initial data may be provided via the camera. Alternatively, or additionally, at least a part of the initial data is provided from those intraoral images which were captured previously, or the preceding intraoral images. Accordingly, the initial data may comprise any one or more of, light intensity measured at a certain location in the oral cavity 106, aperture and/or exposure settings, kinematic data measured via one or more sensors of the intraoral scanner 104, data from previously acquired intraoral images.

The camera is then used to acquire the determined number of intraoral images at or around the location. From at least one of the acquired intraoral images and the initial data or from a plurality of the acquired intraoral images, at least one more optimally exposed two-dimensional intraoral image 122, or a rendered image, is provided. The rendered intraoral image 122 may be provided at the HMI 120, preferably as a plurality of rendered intraoral images 124 such as a video stream or animation.

According to an aspect, the plurality of rendered intraoral images 124 which are captured at different locations in the oral cavity 106 can be used for reconstructing a 3D digital impression 126 of the oral anatomy 108 of the patient 110. The digital impression 126 is any suitable digital representation of the oral anatomy 108, which can comprise natural as well as artificial anatomical features, such as an intraoral scanbody 128 shown in FIG. 1.

In some cases, light is projected on the oral anatomy 108. A plurality of intraoral images of the oral anatomy 108 is generated in response to receiving the reflection. Thus, during the scan, the intraoral scanner 104 is operated to capture the plurality of intraoral images of the oral anatomy 108. The intraoral images may be in the form of a data stream. These intraoral images are provided to the computing module 112. The computing module 112 is configured to provide a plurality of rendered intraoral images 124 from the intraoral images. Advantageously, a data-driven reconstruction logic may be used for reconstructing the plurality of rendered intraoral images 124 from the intraoral images.

The digital impression 126 is usable for manufacturing a dental object such as an aligner, and/or the digital impression 126 may be usable for performing a dental procedure such as a restoration.

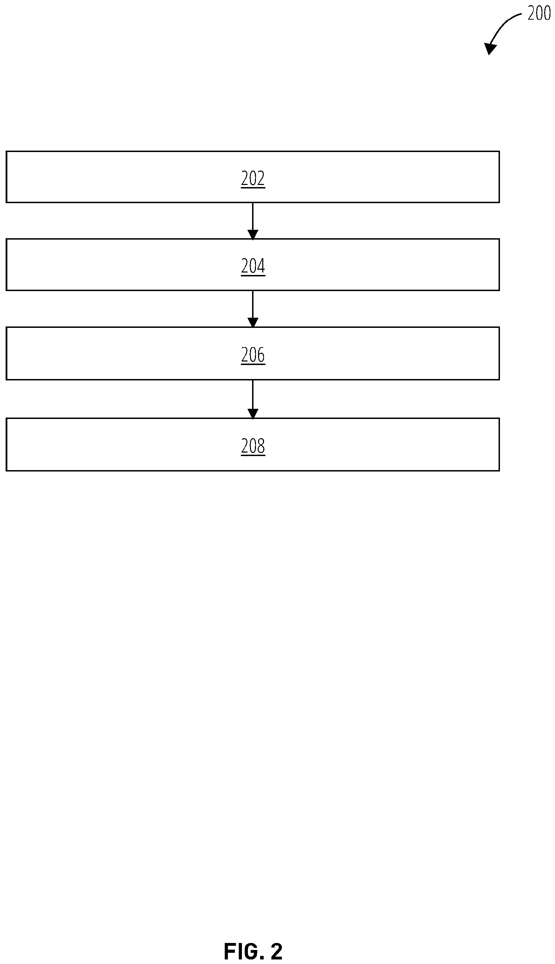

FIG. 2 shows a flowchart 200 which may be implemented as one or more routines which are executed by one or more computing units. At least certain related system aspects shall also be apparent from the following. The flowchart 200 may be implemented as one or more routines in a system such as a dental scanner and/or a scanning system, for example that shown in FIG. 1.

In block 202, it is provided initial data related to: the oral anatomy 108 and/or the intraoral scanner 104.

In block 204, it is computed, using the initial data, a number of intraoral images to be captured via the intraoral scanner 104.

In block 206, it is acquired, via the intraoral scanner 104, said number of intraoral images at a given location at the oral anatomy 108.

In block 208, it is rendered, from at least one of the acquired intraoral images and the initial data or from a plurality of the acquired intraoral images, a rendered intraoral image 122.

The plurality of rendered intraoral images 124 may be used for reconstructing a digital impression 126 of the oral anatomy 108.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for optical intraoral imaging of an oral anatomy via an intraoral scanner, comprising: providing initial data related to: the oral anatomy and/or the intraoral scanner; computing, using the initial data, a number of intraoral images to be captured via the intraoral scanner; acquiring, via the intraoral scanner, said number of intraoral images at a given location at the oral anatomy; rendering, from at least one of the acquired intraoral images and the initial data or from a plurality of the acquired intraoral images, a more optimally exposed two-dimensional intraoral image. The present teachings also relate to systems, software products, uses and storage media. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect.

## Claims

1. A computer-implemented method for optical intraoral imaging of an oral anatomy via an intraoral scanner, which method comprises:
- providing initial data related to: the oral anatomy and/or the intraoral scanner;
- computing, using the initial data, a number of intraoral images to be captured via the intraoral scanner;
- acquiring, via the intraoral scanner, said number of intraoral images at a given location of the oral anatomy;
- rendering, from at least one of the acquired intraoral images and the initial data or from a plurality of the acquired intraoral images, a more optimally exposed two-dimensional intraoral image.

2. Method of claim 1, further comprising:
- providing a plurality of the more optimally exposed two-dimensional intraoral images capturing different locations of the oral anatomy;
- reconstructing, using the plurality of the more optimally exposed two-dimensional intraoral images, a three-dimensional digital impression of the oral anatomy.

3. Method of claim 1 or 2, wherein the initial data comprise any one or more of, light intensity measured at the oral anatomy, aperture and/or exposure settings, kinematic data measured via one or more sensors of the intraoral scanner, data from previously acquired intraoral images.

4. Method of any one of claims 1 to 3, wherein the rendering of the more optimally exposed two-dimensional intraoral image comprises:
- analyzing each of the acquired intraoral images to select one or more regions of desired exposure;
- combining the selected regions to provide the more optimally exposed two-dimensional intraoral image.

5. Method of claim 4, wherein at least some of the regions are selected via a segmentation logic, the segmentation logic preferably being at least partially a data-driven logic.

6. Method of any one of claims 1 to 5, wherein the rendering of the more optimally exposed two-dimensional intraoral image comprises adjusting image properties of at least one of the acquired intraoral images and/or at least one of the selected regions.

7. The method of any one of claims 1 to 6, wherein the rendering of the more optimally exposed two-dimensional intraoral image is at least partially performed via a data-driven rendering logic.

8. Method of any one of claims 1 to 7, wherein image acquisition settings of the intraoral scanner are determined using the initial data.

9. Method of any one of claims 2 to 8, wherein the reconstruction of the three-dimensional digital impression is performed via a data-driven reconstruction logic.

10. A computer software product, or a non-transitory computer-readable storage medium storing the program, comprising instructions which when executed by one or more suitable computing units cause any of the computing unis to perform the steps of any of the above method claims.

11. A system comprising means for performing the steps of any of the above method claims.

12. Use of one or more of the more optimally exposed two-dimensional intraoral images generated in any of the above method claims for reconstructing a digital dental impression and/or display at a human machine interface device and/or training and/or validating a data-driven logic.

13. Use of the three-dimensional digital impression as generated in any one of claims 2 to 9 for performing a dental procedure and/or for manufacture of a dental object.

14. A data-storage medium storing thereupon the three-dimensional digital impression as generated in any one of claims 2 to 9.

15. A data-storage medium storing thereupon one or more of the more optimally exposed two-dimensional intraoral images and/or the digital impression generated in any one of claims 1 to 9.
